# EUROPEAN PATENT SPECIFICATION

(11) **EP 4 409 264 B1**
(45) Date of publication and mention of the grant of the patent: **25.11.2026**
(21) Application number: 22873743.3
(22) Date of filing: 27.09.2022
(51) Int. Cl.: G01N 21/25, G01N 21/31, G01N 21/3504, G01N 33/00, G01J 3/28, E04H 12/18, G01N 21/17, E04H 12/34

(54) **MOBILE SYSTEMS FOR MONITORING EMISSIONS**
MOBILE SYSTEME ZUR EMISSIONSÜBERWACHUNG
SYSTÈMES MOBILES POUR SURVEILLER DES ÉMISSIONS

(30) Priority: 27.09.2021 US 202117485762
(43) Date of publication of application: 07.08.2024
(73) Proprietor: Encino Environmental Services, LLC, Alleyton, Texas 78935 (US)
(72) Inventor: ETHERIDGE, Joe, Alleyton, Texas 78935 (US); KERVICK, Kristopher, Alleyton, Texas 78935 (US)
(74) Representative: Murgitroyd & Company
(86) International application number: PCT/US2022/044905
(87) International publication number: WO 2023/049514

(56) References cited:
- WO-A1-2018/044378
- WO-A1-2020/150388
- CN-B- 103 197 031
- US-A1- 2012 151 852
- US-A1- 2012 151 852
- US-A1- 2016 131 514
- US-A1- 2018 259 418
- US-B1- 10 063 815
- US-B1- 10 063 815
- US-B2- 10 197 470
- RAE SYSTEMS BY HONEYWELL: "AreaRAE Wireless Multi-Gas Monitor - Includes AreaRAE, AreaRAE Gamma, AreaRAE Gamma Steel, And AreaRAE Steel - Operation & Maintenance Manual - Document Number: 029-4034-000 - Revision B, May 2008", 1 May 2008 (2008-05-01), XP002813326, Retrieved from the Internet <URL:https://automation.honeywell.com/content/dam/his-sandbox/products/gas-and-flame-detection/documents/Manual_AreaRAE-All-in-one-Users-Guide_029-4034-000_RevB.pdf> [retrieved on 20250506]
- HAMILTON P. M.: "The application of a pulsed- light rangefinder (lidar) to the study of chimney plumes", PHILOSOPHICAL TRANSACTIONS OF THE ROYAL SOCIETY OF LONDON,SERIES A; MATHEMATICAL AND PHYSICAL SCIENCES., ROYAL SOCIETY, LONDON., GB, vol. 265, no. 1161, GB , pages 153 - 172, XP009545157, ISSN: 0080-4614, Retrieved from the Internet <URL:https://royalsocietypublishing.org/doi/abs110.1098/rsta.1969.0044> DOI: 10.1098/rsta.1969.0044
- ANONYMOUS: "AreaRAE Pro Wireless, transportable area monitor", HONEYWELL, 1 January 2017 (2017-01-01), XP093059725, Retrieved from the Internet <URL:https://sps.honeywell.com/content/dam/his-sandbox/products/gas-and-flame-detection/documents/Brochure_AreaRAE20Pro_DS01173_07-17_LR.pdf> [retrieved on 20230630]

## Description

### FIELD OF THE DISCLOSURE

The present disclosure relates to a mobile system for monitoring emissions such as, for example, methane.

### BACKGROUND AND SUMMARY

Traditional emissions monitoring systems are stationary, complex apparatuses that either intermittently or continuously monitor emissions of gases from, for example, refineries, chemical plants, power plants, or other sources of gaseous emissions. Unfortunately, such traditional systems are immobile and therefore incapable of being moved to, for example, monitor a different location that may be more in need of monitoring. Moreover, such traditional systems are often capable of or configured to only detect one type of emission. In addition, traditional emissions monitoring systems were incapable of processing any data on-site. And traditional systems are often for detecting leaks as opposed to continuous monitoring of low levels of emissions. Thus, use of traditional systems often required transmitting or transporting acquired data to another location for processing and analyzing as to whether remedial action or intervention was required at the source of emission.

What is needed are more effective and cost-efficient systems for monitoring emissions. It would further be advantageous if such systems were mobile, capable of monitoring more than one type of emission, and/or could process data on-site. US10063815B1 discloses a communication vehicle system or platform that provides for the integration of multiple audio, visual, telecommunications, thermal, radiological, chemical and biological data detection and/or communication devices. The communication vehicle can be configured for providing mobile communications and surveillance in an environment in which the surrounding communications and/or power infrastructure has been damaged or is non-existent.. Advantageously, the system described herein may meet one or more up to all of the aforementioned needs whilst improving upon the system or platform disclosed in the prior art.

According to the present invention there is provided a system for monitoring emissions as recited in claim 1.

These and other objects, features and advantages of the exemplary embodiments of the present disclosure will become apparent upon reading the following detailed description of the exemplary embodiments of the present disclosure, when taken in conjunction with the appended claims.

### BRIEF DESCRIPTION OF THE DRAWINGS

Various embodiments of the present disclosure, together with further objects and advantages, may best be understood by reference to the following description taken in conjunction with the accompanying drawings.
Figure 1 shows a trailer with a folded mast and other components of a system for monitoring emissions.
Figure 2 shows a trailer with an extended mast and other components of a system for monitoring emissions.
Figure 3 shows a truck with a folded extendable mast and other components of a system for monitoring emissions.
Figure 4 shows a truck with an extended mast and other components of a system for monitoring emissions.

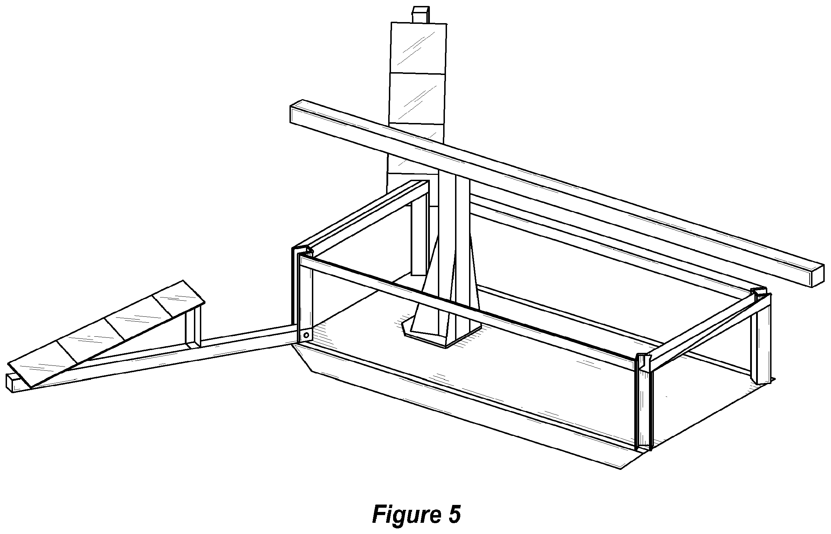

Figure 5 is a front view of a skid mounted unit.

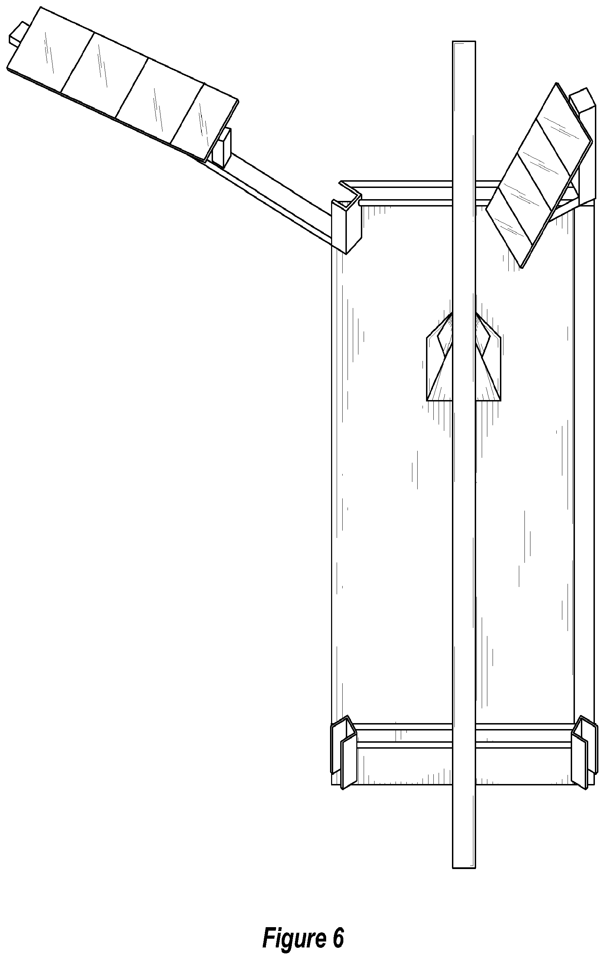

Figure 6 is a top view of a sked mounted unit.

### DETAILED DESCRIPTION

The following description of embodiments provides a non-limiting representative examples referencing numerals to particularly describe features and teachings of different aspects of the invention. The embodiments described should be recognized as capable of implementation separately, or in combination, with other embodiments from the description of the embodiments. A person of ordinary skill in the art reviewing the description of embodiments should be able to learn and understand the different described aspects of the invention. The description of embodiments should facilitate understanding of the invention to such an extent that other implementations, not specifically covered but within the knowledge of a person of skill in the art having read the description of embodiments, would be understood to be consistent with an application of the invention.

The systems for monitoring emissions typically comprise a mobile emission monitoring platform configured to identify and quantify a gas emission; an optional rangefinder configured to determine a size of a gas emission plume and a velocity of the gas emission plume; and an imaging device configured to provide an image. In this manner the system may be configured to continuously monitor a facility emitting an emission.

### Mobile emission monitoring platform

The type of mobile emission monitoring platform is not particularly critical so long as it is capable of identifying and/or quantifying a volume or amount of gas emission of a specific gas or gases to be monitored. Such gases to be monitored by the systems described herein include, for example, a hydrocarbon such as a C1-C6 hydrocarbon like methane or propane, carbon dioxide (CO₂), volatile organic compounds (VOCs), hydrogen sulfide (H₂S), and the like. In some embodiments the mobile emission monitoring platform may comprise a camera that may scan visible, ultraviolet, and/or near-infrared spectral ranges. Such cameras include, for example, hyperspectral cameras configured to identify and/or quantify a volume or amount of gas emission of a specific gas or gases to be monitored. Such cameras may capture a gas or mixture of gases unique infrared absorption to assist in identifying and quantifying an emission. In some embodiments a hyperspectral camera may be configured to identify and quantify two or more up to seven or more different gases. Similarly, in some embodiments the system is configured to identify and quantify a gas emission wherein said gas emission comprises two or more gases or even a mixture of three or more gases.

The emission monitoring platform may be powered by any convenient source. In some embodiments the same power source may power the emission monitoring platform and other components of the system. Such power supplies may include, for example, uninterruptible power supplies. Suitable power supplies include, for example, facility power, a generator, a solar array, etc. In some embodiments, a solar array (as well as accompanying batteries and/or power inverters) may be integral to the unit itself. The emission monitoring platform and/or other components described herein are in some embodiments operably connected to a processor.

### Rangefinder

If desired, a rangefinder may also be included in the systems of the present application. Suitable rangefinders may vary depending upon the other components, the emission, and the desired capability or specificity. Typically, a suitable rangefinder is one configured to determine a size of a gas emission plume and a velocity of the gas emission plume. In some embodiments the rangefinder may facilitate a more accurate measurement of the size of the emission based on its proximity to the camera. As described above for the mobile emission monitoring platform any suitable power source may be employed and the rangefinder may also be operably connected to a processor.

### Imaging device

An imaging device is included in the systems of the present application. Suitable imaging devices may vary depending upon what type of image is desired, e.g., photo, video, and/or combination thereof. The imaging device may also be powered by any suitable power source which source may be the same or different than that for the mobile emission monitoring platform and/or rangefinder. As with the other components described above, the imaging device is operably connected to a processor.

### Other components

The system may include other components which may vary depending upon the desired characteristics of the system. Such components include, for example, a facility interface panel (FIP). A facility interface panel is configured to power and control the system. Components such as a photoionic or other cameras or sensor may be employed. A processor is also employed and operably connected to the emission monitoring platform, the rangefinder, the imaging device, and/or a facility interface panel.

Incorporation of the FIP is an option for the utility and optimization of emissions performance analyses. The Mobile Emissions Platform may be an autonomous system which may operate independently from the system or process to be monitored. However, the FIP may be configured to allow the Mobile Emissions Platform to interface with process-level intelligence systems such as Supervisory Control and Data Acquisition (henceforth "SCADA") systems and Programmable Logic Controllers (henceforth "PLC") which may provide a secondary data layer for determining operational causes of emission activities downstream of each industrial process sensor (e.g., temperature monitor, pressure sensor, and/or flow meter, etc.).

The FIP may be configured to enable the end-user to extract operational insights relating to emissions performance and ultimately provide intelligent feedback which can be used for emissions mitigation and/or process optimization. That is, using the systems and methods described herein the remote sensing platform may be configured to have the ability to communicate with process-level intelligence systems (e.g., drone reconnaissance, manned aircraft reconnaissance, "Picaro" System, optical gas imaging ("OGI") by way of operator/technician) and/or other systems.

The processor may assist in processing data from the various components and may be configured to provide a user with various data points, provide notices of certain conditions, and/or provide signals about corrective actions in regard to an emission. For example, the processor may be configured to provide a notice upon a gas emission above a threshold level or provide a user with an actual emission rate. Additionally or alternatively, a processor may be configured to provide a user with an actual emission rate per activity, an actual emission rate per a piece of equipment, or any combination thereof. In some embodiments the processor may be configured to provide a user with a summary image of an emissions event. Such a summary image may comprise, for example, a hyperspectral image of emission concentration on a visual image of a field of view. A processor may be configured to provide a user with a recorded video of an emissions event. Such a recorded video may comprise an emission concentration a visual image of a field of view. The processor may be configured to provide data such as one or more up to all of the following: hyperspectral data, system telemetry, emission data comprising an emission source, an emission volume, an emission frequency, and/or any combination thereof.

### Remote Sensors

The mobile emissions platform used herein may be configured to accommodate a number of different instruments and technologies. For example, one or more of the following may be incorporated depending upon the needs of the system:

**Ultrasonic gas detection (All Gas Species (sans O₂))**; uses acoustic sensors to identify fluctuations in noise that is imperceptible to the human ear within a process environment. Typically, these sensors have the ability to detect ultrasonic frequencies (25 to 100 KHz), while excluding audible frequencies (0 to 25 KHz). Unlike traditional gas detectors that measure the accumulated gas, ultrasonic gas detectors "hear" the leak, triggering an early warning system.

Ultrasonic technology may be used to detect and measure a gas species where two (2) Mobile Emissions Platforms are utilized; one (1) Mobile Emissions Platform houses an ultrasonic emitter while the second (2) Mobile Emissions Platform houses an ultrasonic transducer (receiver). Gas (target pollutant) is measured through an adiabatic process known as *Time of Flight* ("TOF") where the concentration of the pollutant is determined by the frequency of sound as it travels through an environmental medium (e.g., air).

**Metal Oxide Sensors ("MOS") (Hydrocarbons);** detect concentrations of a gas species by measuring the resistance variation of the metal oxide due to the adsorption gases. Atmospheric oxygen residing on the MOS surface is reduced by target gases, allowing more electrons in the conduction band of the metal oxide material. This resistance (drop) is reversible and varies depending on the reactivity of the sensing materials, presence of catalyst materials, and working temperature of the sensor.

**Non-Dispersive Infrared ("NDIR") through Extractive Interface (Hydrocarbons);** a spectroscopic sensor used in gas detection; is "non-dispersive" in the fact that no dispersive elements (e.g., prism, diffraction gradient; normally present in other types of spectrometers) are used to separate or parse out the broadband light into a narrow spectrum suitable for gas sensing.

The majority of NDIR sensors use a broadband lamp source and an optical filter to select a narrow band spectral region that overlaps with the absorption region of the target gas. Primary components may include:
- Infrared ("IR") source (lamp);
- Sample Chamber or light tube;
- Light filter; and
- IR detector.

**Fourier-Transform Infrared ("FTIR") through Extractive Interface (All Gas Species (sans O₂))**; a technique used to obtain an IR spectrum of absorption or emission of a solid, liquid, or gas. An FTIR spectrometer may simultaneously collect high-resolution spectral data over a wide spectral range. In some cases this may confer a significant advantage over dispersive technology (spectrometer) which measures intensity over a narrow range of wavelengths at a time.

The primary goal of an FTIR system is often to measure how much light a sample absorbs at each wavelength. Rather than introducing a monochromatic beam of light (beam composed of only a single wavelength) to a sample, this technique focuses a beam containing many frequencies of light at once and measures how much of that beam is absorbed by the sample. Next, the beam is modified to contain a different combination of frequencies, providing a second data point. This process is repeated (rapidly) over a short period of time. Afterwards, Fourier Transform (mathematical process) is applied to the raw data set which "reverse analyzes" the spectrum to infer absorption patterns at each wavelength.

The beam is generated with a broadband light source - one (1) containing the full spectrum of wavelengths to be measured. The light is directed through a Michelson interferometer; a certain configuration of mirrors, one (1) of which is moved by a motor. As this mirror moves, each wavelength of light in the beam is periodically blocked, transmitted, blocked, transmitted, by the interferometer, due to wave interference. Different wavelengths are modulated at different rates - so that at each moment or mirror position the beam emitted from the interferometer captures a different spectrum.

**Catalytic Gas Sensor through Extractive Interface (Hydrocarbons);** consisting of a detector element (D) which contains catalytic material and is sensitive to gases, and a compensator element (C) which is inert. Combustible gases will burn only on the detector element, causing a rise in its temperature and, as a consequence, as rise in its resistance. Combustible gases will not burn on the compensator - its temperature and resistance remain unchanged in the presence of combustible gases.

Normally a Wheatstone bridge circuit is formed with both elements. A variable resistor (VR) is adjusted to maintain a state of balance of the bridge circuit in clean-air, free of combustible gases. When combustible gases are present, only the resistance of the detector element will rise, causing an imbalance in the bridge circuit, thus producing an output voltage signal (Vₒᵤₜ). The output voltage signal is proportional to the concentration of combustible gases; gas concentration can be determined by measuring the output voltage.

**Light Detection and Ranging ("LiDAR") (Hydrocarbons);** a method for determining gas concentrations of target species through the use of a tunable laser diode ("TLD"). A TLD utilizes a laser whose wavelength of operation cab be altered in a controlled manner. Since no real laser is truly monochromatic, all lasers can emit light over some range of frequencies, known as linewidth of the laser transition. In most lasers, this linewidth is quite narrow (1,064 nm wavelength transition of a Nd: YAG Laser has a linewidth of approximately 120 GHz or 0.45 nm). Tuning of the laser output across this range can be achieved by placing wavelength-selective optical elements (such as an etalon) into the laser's optical cavity to provide selection of a particular longitudinal mode of the cavity.

The range of applications of tunable lasers is extremely wide. When coupled with the right filter, a tunable source can be configured over a few hundred nanometers with a spectral resolution from 4 nm to 0.3 nm depending on the wavelength range. With proper isolation (>OD4) tunable source can be used for basic absorption and photoluminescence study.

**Photoionization Detection through Extractive Interface (C3+ Hydrocarbons);** measure volatile organic compounds (henceforth "VOC") and other gases in concentrations from sub-parts per billion (ppb) to ten thousand parts per million (10,000 ppm). The photoionization (henceforth "PID") detector is an efficient and inexpensive detector for many gas and vapor analytes.

In a PID, high-energy photons, typically in the vacuum ultraviolet (VUV) range, break molecules into positively-charged ions. As compounds enter the detector they are bombarded by high-energy ultraviolet (UV) photons and are ionized when they absorb the UV light, resulting in ejection of electrons and the formation of positively-charged ions. The ions undergo numerous reactions including reaction with oxygen and/or water vapor, rearrangement, and fragmentation. A few of them may recapture an electron within the detector to reform their original molecules; however, only a small portion of the airborne analytes are ionized (to begin with) so the practical impact of this (if it occurs) is usually negligible. Thus, PIDs are non-destructive and can be used before other sensors in multiple-detector configurations.

The PID will only respond to components that have ionization energies similar to or lower than the energy of the photons produced by the PID lamp. As stand-alone detectors, PIDs are broad band and non-selective, as these may ionize everything with an ionization energy less than or equal to the lamp photon energy. The more common commercial lamps have photons energy upper limits of approximately 8.4 eV, 10.0 eV, 10.6 eV, and 11.7 eV. The major and minor components of clean air all have ionization energies above 12.0 eV and thus do not interfere significantly in the measurement of VOCs, which typically have ionization energies below 12.0 eV.

**Flame-ionization Detection through Extractive Interface (Hydrocarbons);** flame ionization detection ("FID") is a standard instrument used in industry for measuring hydrocarbon gas concentrations; its response is either poor or nil to compounds such as hydrogen sulfide (H₂S), carbon tetrachloride (CCL₄), or ammonia (NH₃). It is mass-sensitive, not concentration sensitive; hence, changes in carrier gas flow rates have little effect on the detector response. It is preferred for general hydrocarbon gas analysis with varying ranges of detection.

The optimum sample gas is introduced into an FID through column input. Any hydrocarbon gas sample will produce ions when burned in the hydrogen flame (located inside the instrument). To detect the ions, two (2) electrodes are used to provide a potential difference. The positive electrode doubles as the nozzle head where the flame is produced. The other electrode is negative, which is positioned above the flame. Thus, the ions are attracted to the negative collector plate - striking a plate which induces a current. This current is proportional to the rate of ionization, which in turn depends upon the concentration of hydrocarbon gas in the sample.

**Electrochemical Gas Detection through Extractive Interface (Combustion** Gases); gas detection systems which measure the concentration of a target gas by oxidizing or reducing the target gas at an electrode and measuring the resulting current.

The gas is extracted and diffuses into a sensor through the back of a porous membrane - directed toward the working electrode where it is oxidized or reduced. The electrochemical reaction results in an electric current that passes through the external circuit. Additionally, the sample is amplified while performing other signal processing functions (from the circuit) while maintaining the voltage across the sensor between the working counter electrodes for a two (2) electrode sensor or between the working and reference electrodes for a three (3) electrode cell. An opposite and equal reaction occurs at the counter electrode, such that if the working electrode results in oxidation, the counter electrode is a reduction.

**Chemiluminescence Gas Analysis through Extractive Interface (Nitrogen Compounds);** operates on the principle that nitric oxide (NO), nitrogen dioxide (NO₂) need to be converted into NO in order to be measured. NO₂ is converted to NO using a molybdenum ("Moly Converter") or stainless-steel converter. Total nitrogen compounds (NOx) is equal to the sum of NO₂ and NO.

Stainless steel is the most popular choice for converter material; however, Moly Converters have been used for years. The stainless-steel converter is heated to six hundred and twenty-five degrees Celsius (625°C) - the Moly Converter is heated to three-hundred and twenty-five degrees Celsius (325°C). The oxide layer of the stainless steel removes one (1) of the oxygen atoms from the NO₂ - resulting in the formation of NO.

The sample flows into the analyzer via a three-way (3-way) solenoid valve. The solenoid typically switches every ten to fifteen seconds (10 sec - 15 sec); the sample is routed either directly into the reaction chamber where only NO is measured or through an N₂O - NO converter where NOx is measured (NO₂ that has been converted to NO and the NO in the sample).

Dry air is pumped in from the ambient environment through a flow switch into an ozone (O₃) generator. The generator creates the O₃ needed for the chemiluminescent reaction. A flow sensor located upstream of the reaction chamber measures the sample flow rate.

In the reaction chamber, the 1-part O₃ reacts with the 2-parts NO in the sample and luminesces. A photomultiplier tube ("PMT") located within a thermoelectric cooler (set a negative three degrees Celsius (-3°C)) - used to eliminate background noise, detects the luminescence generated during the reaction. The sample exhaust exits the reaction chamber through the O₃ converter and is vented to atmosphere.

**Meteorological Station (Contextual Data);** a facility or equipment with instruments used for measuring atmospheric conditions to provide information for weather characteristics and behavior. The measurements include (not limited to):
- Temperature;
- Atmospheric pressure;
- Humidity;
- Wind speed;
- Wind direction; and
- Precipitation.

Wind measurements are taken with as few other obstructions as possible, while temperature and humidity measurements are shielded from direct solar radiation, or insolation. Manual observations are taken periodically; automated measurements are taken at prescribed intervals.

### Extendable mast

According to the invention, the emission monitoring platform, the imaging device and the optional rangefinder and other components are each mounted to an extendable mast. In its extended position in a substantially vertical direction an extendable mast may allow for each component to have a more unobstructed aerial view or line of sight of an emission source. Each of the aforementioned components may be mounted to a separate extendable mast but in most embodiments it is suitable to mount the components to the same extendable mast. In some embodiments at least two up to all of the emission monitoring platform, the rangefinder, and the imaging device are housed within an appliance which appliance is mounted to the extendable mast. Such an appliance protects the individual components from damage due to wind, rain, snow, etc. and in addition substantially aligns the line of sight of the individual components to the emission source or sources of interest. Furthermore, housing the individual components in the appliance facilitates the mounting to the extendable mast.

The extendable mast is configured to be mounted to a vehicle or a trailer for mobility. Such vehicles are not particularly limited and may be a car, truck, semitrailer, boat, or other convenient vehicle depending upon the desired operation. The height of the extendable mast and the location of the appliance on the extendable mast may vary depending upon, for example, the distance and location of the emission source or sources to be monitored. In some embodiments the location of the appliance on the extendable mast be moved up or down via a convenient means such as a winch or an actuator (hydraulic or mechanical). In some embodiments the system is configured such that emissions may be observed from a lateral distance of from about 25 meters up to about 150 meters. In some embodiments the extendable mast is a telescoping mast that extends up to about 40 feet or more in a substantially vertical direction relative to the ground.

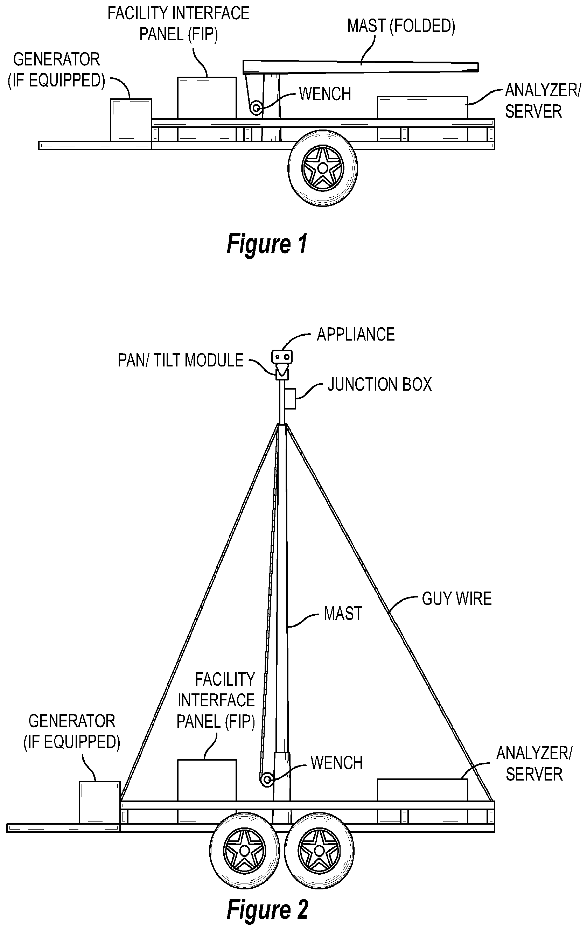

Figure 1 shows a trailer with a folded mast and other components of a system for monitoring emissions. A generator on the trailer may supply power to a facility interface panel that controls the system. A folded telescoping mast may be extended with a winch or other suitable mechanism to a desired height for monitoring emissions. A processor and/or server may analyze and process data locally and be configured to transmit signals to an operator and/or to the emission source being monitored. The appliance with the emission monitoring platform, the rangefinder, and/or the imaging device may be conveniently stored for security when not in operation.

Figure 2 shows the trailer with the attached appliance in an extended position. A remotely operated pan/tilt module provides for precise positioning of the appliance for emission monitoring. A tilt feature advantageously allows the camera to cover a desired area from, or example, the base of the trailer to, for example, the boundary of the camera's detection range.

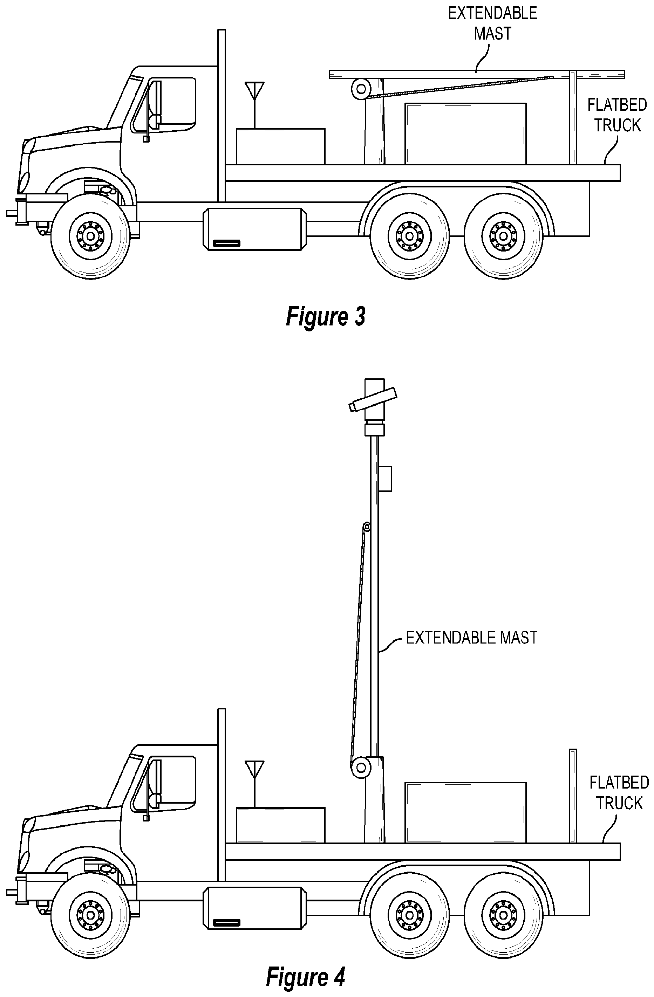

Figure 3 shows a truck with a folded extendable mast and other components of a system for monitoring emissions.

Figure 4 shows a truck with an extended mast and other components of a system for monitoring emissions.

According to the invention as defined in claim 1, the extended mast includes a guy wire. A telescoping or telescopic mast is generally an upright pole or tower that consists of tubular sections that may slide within one another while being extended and/or retracted. Telescopic masts can be made from a variety of materials including, for example, steel, aluminum, other metals, plastics, and combinations thereof depending upon the application, project requirements, or other factors. In some embodiments, the extending telescopic mast may be mounted to or laid adjacent the mobile emissions platform, building, freestanding tripod, skid, or other type of modular device.

The telescoping mast may be utilized in instances where emissions detection capabilities of the instrument are not particularly dependent upon horizontal stability. Alternatively or additionally, the telescoping mast may be reinforced in some convenient manner such that it is horizontally stable. That is, it may be made more rigid and/or stabilized at its base or other mechanism. A telescopic mast may often be appropriate for point-source instruments such as metal oxide detectors, ultrasonic transducers, non-dispersive infrared (NDIR) with extractive interface, and multi-spectral imagers. Telescoping masts may also be useful in areas where overhead electrical hazards are present (and/or piping and industrial process equipment); to avoid contact by limiting the radial divergence from the center of the mast's inflection point (i.e., lay-down mast).

**Adapting the Mobile Emissions Platform to accommodate other Modular Industrial Platforms.**

Advantageously, the systems and methods described herein may be used in lieu of, in addition to, or in partial combination with other modular industrial platforms that may or may not be designed and custom fabricated to operate within specific parameters and/or constraints. Such other modular industrial platforms that may be used with the components and methods described herein include, for example, the following:
**(1) Enclosed utility trailer;** also referred to as a "Cargo Trailer" - a trailer whose cargo area is protected from the elements. Any on-road transportable container sufficiently durable for repeated use which, by virtue of its own particular design, permits the temporary (or permanent) storage and protection of bulk commodities, goods, and other cargo, and which may be transported in various modes without intermediate loading or unloading.
**(2) Self-Contained Cargo Container;** an industrial container or chassis that is rigid, reusable, and capable of being mounted or dismounted/handled by standard container lifting equipment and used by ocean carriers for transportation of goods on board ships; includes any container that is insulated, refrigerated or dry cargo, or described as flat rack, vehicle rack, liquid tank, or open top.
**(3) Skid Mount Unit;** a method of distributing, deploying, and storing machinery and hosting equipment - usually stationary equipment for industrial and scientific purposes; on its own or with other units as part of a modular system. The equipment at the point of manufacture is permanently mounted to a frame, rails, or metal pallet. This equipment can then be easily and readily secured and transported; skid mounted appliances may be temporarily placed within a facility, observation area, warehouse, or onto a vehicle, trailer, or truck. These units often have standard lifting points for maneuverability with a forklift or other lifting device.

In the preceding specification, various embodiments have been described with references to the accompanying drawings. It will, however, be evident that various modifications and changes may be made thereto, and additional embodiments may be implemented, without departing from the scope of the invention as defined by the claims that follow. The specification and drawings are accordingly to be regarded as an illustrative rather than restrictive sense.

## Claims

1. A system for monitoring emissions comprising:
a mobile emission monitoring platform configured to identify and quantify a gas emission wherein the gas emission comprises a hydrocarbon, volatile organic compound, hydrogen sulfide, carbon dioxide, or a mixture thereof;
and
an imaging device configured to provide an image;
wherein the emission monitoring platform and the imaging device are each operably connected to a processor; and
wherein the emission monitoring platform and the imaging device are each mounted to an extendable mast configured to be mounted to a car, a truck, skid, cargo container, or a trailer;
wherein the system further comprises a facility interface panel configured to power and control the system; and
wherein the extendable mast is a telescoping mast with an associated guy wire.

2. The system of claim 1 further comprising a rangefinder configured to determine a size of a gas emission plume and a velocity of the gas emission plume.

3. The system of claim 2 further comprising an appliance mounted to the extendable mast and wherein the appliance houses at least two or more of the emission monitoring platform, the rangefinder, and the imaging device.

4. The system of claim 1 wherein the gas emission comprises methane and the emission monitoring platform comprises a hyperspectral imaging device.

5. The system of claim 1 wherein the processor is configured to provide a notice upon a gas emission above a threshold level.

6. The system of claim 1 wherein the processor is configured to provide a user with an actual emission rate.

7. The system of claim 1 wherein the processor is configured to provide a user with an actual emission rate per activity, an actual emission rate per a piece of equipment, or any combination thereof.

8. The system of claim 1 wherein the processor is configured to provide a user with a summary image of an emissions event wherein said summary image comprises a hyperspectral image of emission concentration on a visual image of a field of view.

9. The system of claim 1 wherein the processor is configured to provide a user with a recorded video of an emissions event wherein said recorded video comprises an emission concentration.

10. The system of claim 1 wherein the processor is configured to provide a user with hyperspectral data and system telemetry; or wherein the processor is configured to provide a user with emission data comprising an emission source, an emission volume, an emission frequency, or any combination thereof.

11. The system of claim 3 wherein the appliance is configured to allow for 360 degree monitoring of a facility emitting an emission.

12. The system of claim 3 wherein the appliance is configured to observe emissions at from about 25 meters up to about 150 meters.

13. The system of claim 1 wherein the system is configured to continuously monitor a facility emitting an emission.

14. The system of claim 1 wherein the emission monitoring platform is configured to identify and quantify a gas emission wherein said gas emission comprises two or more gases.

15. The system of claim 1 wherein the system further comprises a winch for moving the extendable mast up or down.

## Patentansprüche

1. Ein System zur Überwachung von Emissionen, das Folgendes beinhaltet:
eine mobile Emissionsüberwachungsplattform, die konfiguriert ist, um eine Gasemission zu identifizieren und zu quantifizieren, wobei die Gasemission einen Kohlenwasserstoff, eine flüchtige organische Verbindung, Schwefelwasserstoff, Kohlendioxid oder eine Mischung davon beinhaltet;
und
eine Bildgebungsvorrichtung, die konfiguriert ist, um ein Bild bereitzustellen;
wobei die Emissionsüberwachungsplattform und die Bildgebungsvorrichtung jeweils betriebsfähig mit einem Prozessor verbunden sind; und
wobei die Emissionsüberwachungsplattform und die Bildgebungsvorrichtung jeweils an einem ausfahrbaren Mast montiert sind, der konfiguriert ist, um an einem Auto, einem Lastwagen, einem Skid, einem Frachtcontainer oder einem Anhänger montiert zu werden;
wobei das System ferner eine Anlagenschnittstellentafel beinhaltet, die konfiguriert ist, um das System mit Strom zu versorgen und zu steuern; und
wobei der ausfahrbare Mast ein Teleskopmast mit einem assoziierten Abspanndraht ist.

2. System gemäß Anspruch 1, das ferner einen Entfernungsmesser beinhaltet, der konfiguriert ist, um eine Größe einer Gasemissionsfahne und eine Geschwindigkeit der Gasemissionsfahne zu bestimmen.

3. System gemäß Anspruch 2, das ferner ein Gerät beinhaltet, das an dem ausfahrbaren Mast montiert ist, und wobei das Gerät mindestens zwei oder mehr von der Emissionsüberwachungsplattform, dem Entfernungsmesser und der Bildgebungsvorrichtung beherbergt.

4. System gemäß Anspruch 1, wobei die Gasemission Methan beinhaltet und die Emissionsüberwachungsplattform eine hyperspektrale Bildgebungsvorrichtung beinhaltet.

5. System gemäß Anspruch 1, wobei der Prozessor konfiguriert ist, um eine Meldung bei einer Gasemission über einem Schwellenwert bereitzustellen.

6. System gemäß Anspruch 1, wobei der Prozessor konfiguriert ist, um einem Benutzer eine tatsächliche Emissionsrate bereitzustellen.

7. System gemäß Anspruch 1, wobei der Prozessor konfiguriert ist, um einem Benutzer eine tatsächliche Emissionsrate pro Aktivität, eine tatsächliche Emissionsrate pro Ausrüstungsteil oder eine beliebige Kombination davon bereitzustellen.

8. System gemäß Anspruch 1, wobei der Prozessor konfiguriert ist, um einem Benutzer ein zusammenfassendes Bild eines Emissionsereignisses bereitzustellen, wobei das zusammenfassende Bild ein hyperspektrales Bild der Emissionskonzentration auf einem visuellen Bild eines Sichtfelds beinhaltet.

9. System gemäß Anspruch 1, wobei der Prozessor konfiguriert ist, um einem Benutzer ein aufgezeichnetes Video eines Emissionsereignisses bereitzustellen, wobei das aufgezeichnete Video eine Emissionskonzentration beinhaltet.

10. System gemäß Anspruch 1, wobei der Prozessor konfiguriert ist, um einem Benutzer hyperspektrale Daten und Systemtelemetrie bereitzustellen; oder wobei der Prozessor konfiguriert ist, um einem Benutzer Emissionsdaten bereitzustellen, die eine Emissionsquelle, ein Emissionsvolumen, eine Emissionsfrequenz oder eine beliebige Kombination davon beinhalten.

11. System gemäß Anspruch 3, wobei das Gerät konfiguriert ist, um eine 360-Grad-Überwachung einer Anlage, die eine Emission emittiert, zu ermöglichen.

12. System gemäß Anspruch 3, wobei das Gerät konfiguriert ist, um Emissionen bei von etwa 25 Metern bis zu etwa 150 Metern zu beobachten.

13. System gemäß Anspruch 1, wobei das System konfiguriert ist, um eine Anlage, die eine Emission emittiert, kontinuierlich zu überwachen.

14. System gemäß Anspruch 1, wobei die Emissionsüberwachungsplattform konfiguriert ist, um eine Gasemission zu identifizieren und zu quantifizieren, wobei die Gasemission zwei oder mehr Gase beinhaltet.

15. System gemäß Anspruch 1, wobei das System ferner eine Winde zum Auf- oder Abbewegen des ausfahrbaren Masts beinhaltet.

## Revendications

1. Un système pour la surveillance d'émissions comprenant :
une plate-forme de surveillance d'émissions mobile configurée pour identifier et quantifier une émission de gaz où l'émission de gaz comprend un hydrocarbure, un composé organique volatil, du sulfure d'hydrogène, du dioxyde de carbone, ou un mélange de ceux-ci ;
et
un dispositif d'imagerie configuré pour fournir une image ;
où la plate-forme de surveillance d'émissions et le dispositif d'imagerie sont chacun connectés de manière opérationnelle à un processeur ; et
où la plate-forme de surveillance d'émissions et le dispositif d'imagerie sont chacun montés sur un mât extensible configuré pour être monté sur une voiture, un camion, un plateau, un conteneur de fret, ou une remorque ;
où le système comprend en outre un panneau d'interface d'installation configuré pour alimenter et commander le système ; et
où le mât extensible est un mât télescopique avec un câble de hauban associé.

2. Le système de la revendication 1 comprenant en outre un télémètre configuré pour déterminer une taille d'un panache d'émission de gaz et une vitesse du panache d'émission de gaz.

3. Le système de la revendication 2 comprenant en outre un appareil monté sur le mât extensible et où l'appareil loge au moins deux ou plus de deux éléments parmi la plate-forme de surveillance d'émissions, le télémètre, et le dispositif d'imagerie.

4. Le système de la revendication 1 où l'émission de gaz comprend du méthane et la plate-forme de surveillance d'émissions comprend un dispositif d'imagerie hyperspectrale.

5. Le système de la revendication 1 où le processeur est configuré pour fournir une notification lors d'une émission de gaz au-dessus d'un niveau seuil.

6. Le système de la revendication 1 où le processeur est configuré pour fournir à un utilisateur un taux d'émission réel.

7. Le système de la revendication 1 où le processeur est configuré pour fournir à un utilisateur un taux d'émission réel par activité, un taux d'émission réel par équipement, ou toute combinaison de ceux-ci.

8. Le système de la revendication 1 où le processeur est configuré pour fournir à un utilisateur une image récapitulative d'un événement d'émissions où ladite image récapitulative comprend une image hyperspectrale de concentration d'émissions sur une image visuelle d'un champ de vision.

9. Le système de la revendication 1 où le processeur est configuré pour fournir à un utilisateur une vidéo enregistrée d'un événement d'émissions où ladite vidéo enregistrée comprend une concentration d'émissions.

10. Le système de la revendication 1 où le processeur est configuré pour fournir à un utilisateur des données hyperspectrales et une télémétrie système ; ou bien où le processeur est configuré pour fournir à un utilisateur des données d'émission comprenant une source d'émission, un volume d'émission, une fréquence d'émission, ou toute combinaison de ceux-ci.

11. Le système de la revendication 3 où l'appareil est configuré pour permettre une surveillance à 360 degrés d'une installation émettant une émission.

12. Le système de la revendication 3 où l'appareil est configuré pour observer des émissions allant d'environ 25 mètres jusqu'à environ 150 mètres.

13. Le système de la revendication 1 où le système est configuré pour surveiller en continu une installation émettant une émission.

14. Le système de la revendication 1 où la plate-forme de surveillance d'émissions est configurée pour identifier et quantifier une émission de gaz où ladite émission de gaz comprend deux ou plus de deux gaz.

15. Le système de la revendication 1 où le système comprend en outre un treuil pour déplacer le mât extensible vers le haut ou vers le bas.
